# FASCICULE DE BREVET EUROPEEN

(11) **EP 0 869 767 B1**
(45) Date de publication et mention de la délivrance du brevet: **05.09.2001**
(21) Numéro de dépôt: 96944092.4
(22) Date de dépôt: 27.12.1996
(51) Int. Cl.: A61K 7/06, A61K 7/48

(54) **COMPOSITIONS COSMETIQUES POUR LE CHEVEU OU LA PEAU A BASE DE COPOLYESTERS SULFONES A MOTIFS POLYORGANOSILOXANES**
KOSMETISCHE HAAR-ODER HAUTZUBEREITUNGEN AUF BASIS VON SULFONIERTEN COPOLYESTERN MIT POLYORGANOSILOXAN EINHEITEN
COSMETIC COMPOSITIONS FOR THE HAIR OR SKIN BASED ON SULFONE COPOLYESTERS WITH POLYORGANOSILOXANE UNITS

(30) Priorité: 29.12.1995 FR 9515713
(43) Date de publication de la demande: 14.10.1998
(73) Titulaire: RHODIA CHIMIE, 92408 Courbevoie Cédex (FR)
(72) Inventeur: FLEURY, Etienne, F-69540 Irigny (FR); RICCA, Jean-Marc, F-75012 Paris (FR)
(74) Mandataire: Fabre, Madeleine-France
(86) Numéro de dépôt international: FR9602092
(87) Numéro de publication internationale: WO9724104

(56) Documents cités:
- US-A- 5 320 836

## Description

La présente invention a pour objet des compositions cosmétiques destinées à être utilisées sur le cheveu et/ou la peau. Elles sont à base de copolyesters sulfonés à motifs polyorganosiloxanes ; elles peuvent contenir différents autres ingrédients, notamment des agents conditionneurs pour le cheveu et/ou la peau. Ces compositions, de par leur application externe sur la peau, peuvent être en outre utilisées pour transporter et/ou délivrer à travers la peau un certain nombre de produits actifs cosmétiques ou pharmaceutiques.

On entend par le terme composition ou formulation cosmétique tous les produits ou préparations cosmétiques comme celles décrites dans l'annexe I ("illustrative list by category of cosmetic products") de la directive européenne n° 76/768/CEE du 27 juillet 1976 dite directive cosmétique.

La Demanderesse a trouvé que certains copolyesters sulfonés à motifs polyorganosiloxanes sont particulièrement aptes à être utilisés dans les compositions cosmétiques pour le cheveu ou la peau.

La présente invention a pour objet une composition cosmétique pour le cheveu et/ou la peau, caractérisée en ce qu'elle comprend au moins un copolyester sulfoné hydrodispersable à motifs polyorganosiloxanes comportant une pluralité de motifs récurrents polyesters sulfonés et de motifs polyorganosiloxanes, ledit copolyester sulfoné à motifs polyorganosiloxanes présentant
. une masse moléculaire en nombre de l'ordre de 5 000 à 45 000, de préférence de l'ordre de 8 000 à 35 000,
. une teneur pondérale en soufre de l'ordre de 0,5 à 10 %, de préférence de l'ordre de 1 à 8 %, par rapport auxdits copolyesters sulfonés à motifs polyorganosiloxanes
. et une teneur en silicium de l'ordre de 0,05 à 20 %, de préférence de l'ordre de 0,1 à 10 %, tout particulièrement de l'ordre de 0,1 à 5 % en poids par rapport auxdits copolyesters sulfonés à motifs polyorganosiloxanes.

Ledit copolyesters sulfoné hydrodispersable à motifs polyorganosiloxanes, copolyesters est tout particulièrement choisi parmi ceux susceptibles d'être obtenus par polymérisation (estérification et/ou transestérification et polycondensation) d'une composition monomère (M) à base :
- d'au moins un monomère acide dicarboxylique aromatique non sulfoné (A), son anhydride ou un de ses diesters,
- d'au moins un monomère diol aliphatique ou cycloaliphatique (D),
- d'au moins un monomère sulfoné (MS) choisi parmi les acides dicarboxyliques aromatiques sulfonés ou aliphatiques sulfonés (MAS), leurs anhydrides ou leurs diesters et les diols aliphatiques sulfonés ou aromatiques sulfonés (MDS), les quantités relatives des monomères, sulfonés ou non, à fonctions diacides, diesters ou anhydride, et des monomères, sulfonés ou non, à fonctions diols, correspondant à un rapport nombre de fonctions OH de la composition monomère / nombre de fonctions ou d'équivalents fonctions COOH de la composition monomère de l'ordre de 1,05 à 4, de préférence de l'ordre de 1,1 à 3,5, tout particulièrement de l'ordre de 1,8 à 3 ; ladite opération de polymérisation étant réalisée en présence d'au moins un polydiorganosiloxane réactif de formule (I) 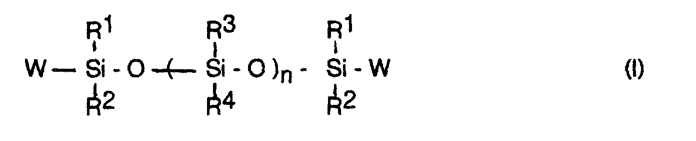 formule dans laquelle
- les symboles R¹, R², R³ et R⁴, identiques ou différents, représentent des radicaux alkyles linéaires ou ramifiés en C₁-C₁₆, de préférence en C₁-C₄, cycloalkyles en C₅-C₁₅, de préférence en C₆-C₈, phényle, alkylphényle avec une partie alkyle en C₁-C₄,
- W représente un groupement susceptible de réagir avec au moins un des monomères de la composition monomère (M) dans les conditions de l'opération de polymérisation,
- n est un nombre entier ou décimal de l'ordre de 5 à 15 000, de préference de l'ordre de 30 à 300, et tout particulièrement de l'ordre de 50 à 200 ;
les quantités de monomère sulfoné (MS) et de polyorganosiloxane, ainsi que les conditions de polymérisation étant telles que lesdits copolyesters sulfonés à motifs polyorganosiloxanes obtenus présentent
. une masse moléculaire en nombre de l'ordre de 5 000 à 45 000, de préférence de l'ordre de 8 000 à 35 000,
. une teneur pondérale en soufre de l'ordre de 0,5 à 10 %, de préférence de l'ordre de 1 à 8 %, par rapport auxdits copolyesters sulfonés à motifs polyorganosiloxanes,
. et une teneur en silicium de l'ordre de 0,05 à 20 %, de préférence de l'ordre de 0,1 à 10 %, tout particulièrement de l'ordre de 0,1 à 5 % en poids par rapport auxdits copolyesters sulfonés à motifs polyorganosiloxanes.

Les masses moléculaires en nombre sont mésurées par chromatographie par perméation de gel, dans le diméthylacétamide contenant 10⁻² N de LiBr, à 25°C. Les résultats sont exprimés en équivalents polystyrène.

Les monomères acides dicarboxyliques aromatiques non sulfoné (A), sont de préférence choisis parmi les acides téréphtalique, isophtalique et 2,6 naphtalène dicarboxylique, leurs diesters ou leur anhydride.

Le monomère diacide non sulfoné (A) est de préférence constitué par de l'ordre de 0 à 100% molaire, de préférence de l'ordre de 50 à 100% molaire, tout particulièrement de l'ordre de 70 à 90% molaire, d'acide téréphtalique et/ou isophtalique et/ou 2,6-naphtalène dicarboxylique sous forme d'un de ses (leurs) diesters inférieurs de méthyle, éthyle, propyle, isopropyle ou butyle et de l'ordre de 100 à 0% molaire, de préférence de l'ordre de 50 à 0% molaire, tout particulièrement de l'ordre de 30 à 10% molaire, d'acide ou anhydride isophtalique et/ou 2,6-naphtalène dicarboxylique et/ou terephtalique.

Ledit monomère non sulfoné (A) peut être tout particulièrement constitué par 50 à 90% molaire, tout particulièrement de l'ordre de 70 à 90% molaire, d'acide téréphtalique sous forme d'un de ses (leurs) diesters inférieurs de méthyle, éthyle, propyle, isopropyle, ou butyle et de l'ordre de 50 à 10% molaire, de préférence de l'ordre de 30 à 10% molaire, d'acide ou anhydride isophtalique .
Peuvent en outre être présentes, à côté de ces acides dicarboxyliques (anhydrides ou diesters), des quantités mineures de diacides aromatiques autres tels que l'acide orthophtalique, les acides anthracène, 1,8-naphtalène, 1,4-naphtalène, biphenyl dicarboxyliques, ou de diacides aliphatiques tels que les acides adipique, glutarique, succinique, triméthyladipique, pimélique, azelaique, sebacique, suberique, itaconique, maleique ... sous forme acide, anhydride ou diesters inférieurs (de méthyle, éthyle, propyle, isopropyle, butyle).

Parmi les monomères diols (D) aliphatiques ou cycloaliphatiques, on peut citer les oxyalkylène glycols aliphatiques contenant de 1 à 100 motifs oxyalkylènes, tels que l'éthylène glycol, le propylène glycol, le diéthylène glycol, le dipropylène glycol et leurs homologues supérieurs, le butanediol-1,4, l'hexanediol-1,6, le neopentylglycol et les glycols cyclaniques, tels que le cyclohexanediol, le cyclohexane diméthanol, le dicyclohexanediolpropane ... ; le monomère diol (D) préférentiellement mis en oeuvre est le monoéthylène glycol et/ou le diéthylène glycol.

Le monomère sulfoné (MS) est de préférence constitué d'un acide dicarboxylique sulfoné (MAS), celui-ci présentant au moins un groupe acide sulfonique, de préférence sous la forme d'un sulfonate de métal alcalin (de sodium en particulier), alcalino-terreux, d'ammonium , d'ammonium quaternaire ..., et deux fonctions acides fixées sur un ou plusieurs cycles aromatiques, lorsqu'il s'agit d'acides dicarboxyliques aromatiques, ou sur la chaîne aliphatique, lorsqu'il s'agit d'acides dicarboxyliques aliphatiques.
Parmi les monomères sulfonés (MS), on peut citer les acides ou anhydrides dicarboxyliques sulfonés aromatiques tels que les acides ou anhydrides sulfo-isophtaliques, sulfo-terephtaliques, sulfo-orthophtaliques, les acides ou anhydrides sulfo-4 naphtalène dicarboxylique-2,7, les sulfo-bis(hydroxycarbonyl)-4,4' diphénylsulfones, les acides ou anhydrides sulfo-diphényl-dicarboxyliques, les sulfo-bis(hydroxycarbonyl)-4,4' diphénylméthanes, les acides ou anhydrides sulfo-phénoxy-5 isophtaliques ou leurs diesters inférieurs (de méthyle, éthyle, propyle, isopropyle, butyle).et les acides ou anhydrides dicarboxyliques sulfonés aliphatiques tels que les acides ou anhydrides sulfo-succiniques ou leurs diesters inférieurs (de méthyle, éthyle, propyle, isopropyle, butyle). Les monomères sulfonés préférentiels sont les acides ou anhydrides sulfo-isophtaliques et sulfo-succiniques et leur diester de méthyle, et tout particulièrement le sodio-oxysulfonyl-5 isophtalate de diméthyle.
Ledit monomère diacide sulfoné (MAS) peut être mis en oeuvre en quantité correspondant à un rapport molaire (MAS) / (A)+(MAS) de l'ordre de 5/100 à 40/100, de préférence de l'ordre de 7/100 à 15/100, tout particulièrement de l'ordre de 10/100 à 15/100.

L'entité élémentaire considérée dans la définition de la mole de monomère (A) ou ou (MAS) est la fonction COOH dans le cas des diacides ou l'équivalent fonction COOH dans le cas de l'anhydride ou des diesters.

Les polydiorganosiloxanes réactifs pouvant être mis en oeuvre selon l'invention, sont préférentiellement ceux de formule (I), dans laquelle W représente une fonction OH ou un groupement -A-Z, où :
. A représente un radical hydrocarboné divalent lié à l'atome de silicium vicinal par une liaison Si-C ou Si-O-C, pouvant éventuellement comporter un hétéroatome pris dans le groupe formé par O, Si, N et S
. Z représente un groupement susceptible de réagir avec au moins un des monomères ci-dessus dans les conditions de polymérisation.
Parmi les radicaux R¹, R², R³ et R⁴, identiques ou différents, prérérentiels, on peut citer les radicaux méthyle, éthyle, n-propyle, isopropyle, n- butyle, isobutyle,n- pentyle, éthyl-2 hexyle, n-décyle ; cyclopentyle, cyclohexyle ; phényle ; le radical méthyle étant tout particulièrement préféré.
Parmi les radicaux divalents A, on peut citer les groupes
* alkylènes en C₁-C₁₅, de préférence en C₁-C₁₂ tels que méthylène, éthylène, propylène-1,3, butylène-1,4, alpha-méthylpropylène-1,3 , pentylène-1,5 , hexaméthylène, décaméthylène
* arylènes ou polyaryiènes tels que phénylène, diphénylène, naphtylène, éventuellement subsitués par un ou plusieurs radicaux alkyles en C₁-C₄
* arylènes-alkylènes constitués d'un enchaînement de 1 ou plusieurs radicaux arylènes [o, m ou p-phénylène(s) éventuellement substitué(s) par un ou plusieurs radicaux alkyle(s) en C₁-C₄], et d'un ou plusieurs radicaux alkylène(s) en C₁-C₁₅
* alkylènes-arylènes constitués d'un radical alkylène en C₁-C₄ et d'un ou plusieurs radicaux arylène(s), phénylène(s) notamment
* alkylènes, arylènes, polyarylènes ou arylènes-alkylènes, alkylènes-arylènes tels que ci-dessus, comportant en outre 1 ou plusieurs hétéroatome choisi parmi O, Si, N et S tels que polyoxyalkylène, phénylèneoxyalkylène ...
Parmi les groupements Z réactifs on peut mentionner les groupements OH, NH₂, NCO, COOH, COOX où X représente un atome d'halogène (chlore, brome), COOR, où R représente ou un reste alkyle linéaire ou ramifié en C₁-C₈, de préférence méthyle.

A titre d'exemple de motifs réactifs W on peut citer

| | |
|---|---|
| -OH | |
| -(CH₂)₂-OH | -(CH₂)₃-OH |
| -(CH₂)₂-O-(CH₂)₂-OH | -(CH₂)₃-O-Φ-OH |
| -(CH₂)₂-COOCH₃ | -Φ -COOCH₃ |
| -(CH₂)₃-Φ-COOCH₃ | -(CH₂)₃-O-Φ-COOCH₃ |
| -(CH₂)₃-O-(CH₂)₂-OH | -(CH₂)₃-O-CH₂-CH(CH₃)OH |

Ces polyorganosiloxanes réactifs sont des produits de types connus ; ils peuvent être préparés par les voies usuelles de synthèse des polyorganosiloxanes di fonctionnels ; par exemple, ils peuvent être obtenus par réaction d'un polyorganohydrogénosiloxane avec un composé organique fonctionnel à insaturation éthylénique, en présence d'un dérivé du platine, ou encore par réaction d'un organodialkylmonochlorosilane à groupe fonctionnel avec un polyorganosiloxanediol ou un de ses éthers d'alkyle inférieur.

Un mode de préparation de copolyesters sulfonés à motifs polysiloxanes du type de ceux décrits ci-dessus, a déjà été mentionné dans la demande de brevet EP-A-430828.

Les copolyesters sulfonés à motifs polysiloxanes décrits ci-dessus, peuvent être obtenus par les procédés usuels d'esterification et/ou transestérification et polycondensation, le polyorganosiloxane pouvant être introduit à un moment quelconque de l'opération d'esterification et/ou transestérification.

Ainsi lesdits copolyesters sulfonés à motifs polysiloxanes peuvent être obtenus en réalisant les étapes suivantes :
- une étape d'esterification et/ ou transestérification entre les acides dicarboxyliques sulfonés ou non, leurs diesters ou anhydrides, et les diols sulfonés ou non , le rapport nombre de fonctions OH de la composition monomère / nombre de fonctions ou équivalents fonctions COOH de la composition monomère étant de l'ordre de 1,05 , à 4, de préférence de l'ordre de 1,1 à 3,5, tout particulièrement de l'ordre de 1,8 à 3
   ledit polyorganosiloxane étant introduit à un moment quelconque de cette étape,
- et une étape de polycondensation.

L'étape d'estérification et/ou transestérification peut être effectuée à une température supérieure ou égale à 130°C, de préférence de l'ordre de 140 à 220°C et tout particulièrement de l'ordre de 180 à 220°C . Cette opération d'estérification et/ou transestérification est de préférence réalisée en présence d'un catalyseur d'estérification ou de transestérification métallique, notamment d'un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2"-triéthyle (ou aminotriéthanolate de titane) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de monomères présents.
La durée de l'opération d'estérification et/ou transestérification est de l'ordre de 1 à 10 heures.
La réaction s'effectue avec formation d'eau et éventuellement d'un alcool (méthanol dans le cas de la mise en oeuvre d'au moins un acide dicarboxylique sulfoné ou non sous la forme d'un diester méthylique), qui sont éliminés du milieu réactionnel de préférence par distillation en même temps que le diol en excès.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression fonction de la masse moléculaire désirée.Le vide est ensuite cassé à l'azote, et le polymère est coulé dans une lingotière ; après refroidissement, le polymère est broyé.

Les copolyesters sulfonés à motifs polysiloxanes préférentiels sont ceux susceptibles d'être obtenus à partir
- d'acide isophtalique ou terephtalique (A1) et/ou d'un diester de l'acide téréphtalique ou isophtalique (A2), selon un rapport molaire (A1)/(A2) de l'ordre de 0/100 à 100/0, de préférence de l'ordre de 0/100 à 50/50, tout particulièrement de l'ordre de 10/90 à 30/70, (A2) se trouvant de préférence sous forme de diester de méthyle,
- d'un diester de l'acide sulfoisophtalique (MAS), de préférence sous forme de diester de méthyle, selon un rapport molaire (MAS)/(A1)+(A2)+(MAS) de l'ordre de 5/100 à 40/100, de préférence de l'ordre de 7/100 à 35/100, tout particulièrement de l'ordre de 10/100 à 15/100.
- de monoéthylène glycol et/ou de diéthylèneglycol (D), selon un rapport nombre de fonctions OH de (D) / nombre de fonctions ou équivalents fonctions COOH de (A1)+(A2)+(MAS) de l'ordre de 1,05 à 4, de préférence de l'ordre de 1,1 à 3,5, tout particulièrement de l'ordre de 1,8 à 3
- d'un polyorganosiloxane de formule (I) en quantité telle que ledit copolyester sulfoné à motifs polyorganosiloxane présente
   . une teneur pondérale en soufre de l'ordre de 0,5 à 10 %, de préférence de l'ordre de 1 à 8 %, par rapport auxdits copolyesters sulfonés à motifs polyorganosiloxanes,
   . et une teneur en silicium de l'ordre de 0,05 à 20 %, de préférence de l'ordre de 0,1 à 10 %, tout particulièrement de l'ordre de 0,1 à 5 % en poids par rapport auxdits copolyesters sulfonés à motifs polyorganosiloxanes.

Des copolyesters sulfonés à motifs polyorganosiloxanes tout particulièrement intéressants, sont ceux susceptibles d'être obtenus à partir
- d'acide isophtalique (A1) et d'un diester de l'acide téréphtalique (A2), selon un rapport molaire (A1)/(A2) de l'ordre de 10/90 à 50/50, de préférence de l'ordre de 10/90 à 30/70, (A2) se trouvant de préférence sous forme de diester de méthyle,
- d'un diester de l'acide sulfoisophtalique (MAS), de préférence sous forme de diester de méthyle, selon un rapport molaire (MAS)/(A1)+(A2)+(MAS) de l'ordre de 5/100 à 40/100, de préférence de l'ordre de 7/100 à 35/100, tout particulièrement de l'ordre de 10/100 à 15/100
- de monoéthylène glycol et/ou de diéthylèneglycol (D), selon un rapport nombre de fonctions OH de (D) / nombre de fonctions ou équivalents fonctions COOH de (A1)+(A2)+(MAS) de l'ordre de 1,05 à 4, de préférence de l'ordre de 1,1 à 3,5, tout particulièrement de l'ordre de 1,8 à 3
- d'un polyorganosiloxane de formule (I) en quantité telle que ledit copolyester sulfoné à motifs polyoirganosiloxane présente
   . une teneur pondérale en soufre de l'ordre de 0,5 à 10 %, de préférence de l'ordre de 1 à 8 %, par rapport auxdits copolyesters sulfonés à motifs polyorganosiloxanes,
   . et une teneur en silicium de l'ordre de 0,05 à 20 %, de préférence de l'ordre de 0,1 à 10 %, tout particulièrement de l'ordre de 0,1 à 5 % en poids par rapport auxdits copolyesters sulfonés à motifs polyorganosiloxanes.

Ces copolyesters sulfonés à motifs polyorganosiloxanes préférentiels ou particuliers peuvent être préparés selon un procédé préférentiel par réalisation des étapes successives suivantes :
- une étape de transestérification entre d'une part le diester de l'acide téréphtalique ou isophtalique (A2) et le diester de l'acide sulfoisophtalique (MAS) et d'autre part du monoéthylène glycol et/ou le diéthylèneglycol (D), le rapport nombre de fonctions OH de (D) / nombre de fonctions COOH de (A2)+(MAS) étant de l'ordre de 1,05 à 4, de préférence de l'ordre de 1,1 à 3,5, tout particulièrement de l'ordre de 1,8 à 3, les diesters (A2) et (MAS) étant de préférence des diesters de méthyle
- une étape d'estérification entre l'acide isophtalique ou terephtalique (A1) lorsqu'il est présent et d'autre part le monoéthylène glycol et/ou le diéthylène glycol (D), le rapport nombre de fonctions OH de (D) / nombre de fonctions COOH de (A1) étant de l'ordre de 1,05 à 4, de préférence de l'ordre de 1,1 à 3,5, tout particulièrement de l'ordre de 1,8 à 3,
- une étape de polycondensation
ledit polyorganosiloxane de formule (I) étant introduit soit à l'étape de transestérifiaction, soit à l'étape d'estérification, et de préférence à l'étape d'estérification dans le milieu d'estérification.

L'étape de transestérification est effectuée en présence d'un catalyseur de transestérification métallique à une température supérieure ou égale à 130°C, de préférence de l'ordre 140 à 220°C et tout particulièrement de l'ordre de 180 à 220°C ; à cette température le méthanol formé est éliminé du milieu réactionnel de préférence par distillation. La durée de cette opération est de l'ordre de 1 à 4 heures, et généralement de l'ordre de 2 à 3 heures.
Lorsque plus de 90% de la quantité théorique de méthanol a été distillée, le polyol excédentaire est éliminé en portant la température du milieu réactionnel à 230°C.

L'opération d'estérification est réalisée par ajout dans le milieu réactionnel, de l'acide dicarboxylique aromatique non sulfoné et de la fraction restante de diol (D), préalablement mis en suspension, à une température correspondant à celle de la fin de la température de transestérification; la période d'introduction est de l'ordre de 1 heure. Cette opération d'estérification est réalisée à une température de l'ordre de 230 à 280°C, de préférence de l'ordre de 250 à 260°C, en présence d'un catalyseur du même type que celui de transestérification ; la réaction s'effectue avec élimination d'eau qui est soutirée du réacteur en même temps que le diol en excès.
Le polyorganosiloxane de formule (I) est introduit de préférence à l'étape d'estérification dans le milieu d'estérification.

Le copolyester sulfoné à motifs polyorganosiloxanes ainsi obtenu peut être dispersé ou solubilisé dans l'eau déminéralisée jusqu'à obtenir un taux d'extrait sec de l'ordre de 3 à 30%, de préférence de l'ordre de 7 à 15% en poids ; cette opération de dispersion ou de solubilisation est de préférence réalisée sous agitation, à une température de l'ordre de 60 à 80°C.

Les quantités de copolyesters sulfonés à motifs polyorganosiloxanes pouvant être présentes dans les compositions cosmétiques de l'invention peuvent représenter de 0,1 à 50%, de préférence de l'ordre de 0,1 à 5% du poids desdites compositions cosmétiques.

Le copolyester sulfoné à motifs polyorganosiloxanes peut être formulé en un grand nombre de compositions cosmétiques pour le cheveu et/ou la peau, comme des formules de rinçage, des lotions, des shampoings, des conditionneurs, des mousses, des gels coiffants ou toute autre formulation pour le coiffage ou pour faciliter le peignage des cheveux, ainsi que les lotions pour les mains ou le corps, les laits de toilette, les compositions démaquillantes, les produits régulant l'hydratation de la peau, les crèmes de soin, les crèmes ou laits de protection contre le soleil et le rayonnement ultra-violet, les préparations anti-acnée, les analgésiques locaux, les mascaras ; il peut être également utilisé comme constituant de compositions solides compactes et homogènes comme les pains de toilette, les savonnettes.

Ledit copolyester sulfoné à motifs polyorganosiloxanes est présent dans la composition cosmétique au sein d'un véhicule compatible avec le cheveu et/ou la peau dont la fonction est d'entraîner ledit copolyester à motifs polyorganosiloxanes sur le cheveu et/ou la peau au moment de son application. Ce véhicule peut représenter de 0,5 à 99,5%, de préférence de 5 à 99,5 % du poids de la composition cosmétique.

La présente invention a donc également pour objet une composition cosmétique comprenant :
- de 0,1 à 50 %, de préférence de 0,1 à 5 % en poids du copolyester sulfoné à motifs polyorganosiloxanes ci-dessus décrit
- et de 0,5 à 99,5%, de préférence de 5 à 99,5% en poids d'un véhicule compatible avec le cheveu et/ou la peau.
Le terme "compatible avec une application sur le cheveu et/ou la peau" signifie içi que le véhicule n'abime pas ou n'exerce pas d'effets négatifs sur l'aspect du cheveu et/ou de la peau ou ne crée pas d'irritation de la peau et/ou l'oeil et/ou le cuir chevelu.
Les véhicules compatibles avec les formulations décrites dans cette invention comprennent par exemple ceux utilisés dans les sprays, les mousses, les toniques, les gels, les shampoings, ou les lotions de rinçage. Le choix du véhicule approprié dépendra de la nature du copolyester à motifs polyorganosiloxanes utilisé, et de sa destination, selon que le produit formulé est censé être laissé sur la surface où il a été appliqué (par exemple sprays, mousses, lotion tonique, ou gels) ou rincé après utilisation (par exemple shampoing, conditionneur, lotions de rinçage).
Les véhicules susceptibles d'être utilisés peuvent donc être simples ou complexes, inclure un grand nombre de produits habituellement utilisés dans les compositions cosmétiques pour le cheveu et/ou la peau. Le véhicule peut être de l'eau contenant éventuellement un solvant pour solubiliser ou disperser le copolymère utilisé, tel que les alcools en C1-C6, et leurs mélanges, en particulier méthanol, éthanol, isopropanol, et leurs mélanges. Le véhicule peut aussi contenir une grande variété d'autres composés comme l'acétone, des hydrocarbures (comme l'isobutane, l'hexane, le décène), des hydrocarbures halogénés (comme les fréons), le linalol, des esters (comme l'acétate d'éthyle, le phtalate de dibutyle), et des silicones volatils (en particulier des siloxanes comme le phényl pentaméthyl siloxane, le méthoxypropyl heptaméthyl cyclotétrasiloxane, le chloropropyl pentaméthyl disiloxane, l'hydropropyl pentaméthyl disiloxane, l'octaméthyl cyclotétrasiloxane, le décaméthyl cyclopenta siloxane, la cyclodiméthicone, et la diméthicone), et leurs mélanges. Lorsque les compositions cosmétiques se présentent sous la forme de sprays, lotions toniques, gels, ou mousses, les solvants préférentiels comprennent l'eau, l'éthanol, les dérivés volatils de silicone, et leurs mélanges. Les solvants utilisés dans ces mélanges peuvent être miscibles ou non miscibles les uns avec les autres. Les mousses et les sprays aérosol peuvent aussi utiliser n'importe quel propulseur capable de générer les produits sous forme de mousse ou de sprays fins, uniformes. A titre d'exemples, on peut citer le trichlorofluorométhane, le dichlorodifluorométhane, le difluoroéthane, le diméthyléther, le propane, le n-butane, ou l'isobutane.
Dans le cas où ces compositions cosmétiques sont utilisées pour une application locale sur la peau, les véhicules doivent avoir de bonnes propriétés esthétiques, être compatibles avec les copolyesters à motifs polyorganosiloxanes et tous les autres composants, et ne pas poser de problèmes concernant la toxicité et l'irritation.
Ces véhicules peuvent prendre un grand nombre de formes, par exemple émulsion, mousses, sprays .... Par exemple, les véhicules sous forme d'émulsions incluent les émulsions eau dans huile, huile dans eau, et huile dans eau dans silicone. Ces émulsions couvrent une grande plage de viscosité, par exemple de 100 à 200000 mPa.s à 25°C. Ces émulsions peuvent aussi être délivrées sous forme de sprays en utilisant soit un dispositif de type pompe mécanique, soit sous forme d'aérosol préssurisé par l'emploi d'un gaz propulseur.
Ces véhicules peuvent aussi être délivrés sous forme de mousse.
On peut citer par exemple les solvants liquides anhydres, comme les huiles, les alcools et les silicones, les mélanges aqueux homogènes comme les mélanges hydroalcooliques, et les versions rhéologiquement modifiées de ces deux systèmes, par exemple quand la viscosité du système a été augmentée par l'addition de gommes, résines, polymères, ou sels).

Les compositions cosmétiques faisant l'objet de l'invention peuvent également contenir d'autres additifs.
Elles peuvent notamment contenir des résines fixatives dont la structure ne contient pas de motifs silicone.
Ces résines fixatives sont préférentiellement dispersées ou solubilisées dans le véhicule choisi avec le copolymère silicone. Ces résines fixatives peuvent être de nature anionique, cationique, non ionique, amphotère, ou un mélange de ces différentes natures. Celles-ci sont préférentiellement de nature anionique ou amphotère.
Ces résines fixatives sont généralement présentes dans les compositions cosmétiques à des concentrations comprises entre 0.5 et 10%, préférentiellement entre 1 et 5%.
Elles sont préférentiellement choisies parmi les résines suivantes : copolymères acrylate de méthyle / acrylamide, copolymères polyvinylméthyléther / anhydride maléique, copolymères acétate de vinyle / acide crotonique, copolymères octylacrylamide / acrylate de méthyle / butylaminoéthylméthacrylate, polyvinylpyrrolidones, copolymères polyvinylpyrrolidone / méthacrylale de méthyle, copolymères polyvinylpyrrolidone */* acétate de vinyle, alcools polyvinyliques, copolymères alcool polyvinylique / acide crotonique, copolymères alcool polyvinylique / anhydride maléique, hydroxypropyl celluloses, hydroypropyl guars, polystyrène sulfonates de sodium, terpolymères polyvinylpyrrolidone / éthyl méthacrylate/ acide méthacrylique, monométhyl éthers de poly(méthylvinyl éther / acide maléique), les copolymères polytéréphtale d'éthylène glycol / polyéthylène glycol, les copolymères polytéréphtalate d'éthylène glycol / polyéthylène glycol / polyisophtalate sulfonate de sodium, et leurs mélanges.
De plus, des résines cationiques peuvent également être utilisées. Ces résines cationiques dérivent en tout ou partie de monomères cationiques comme par exemple le méthacrylate de diméthylaminoéthyle, le méthacrylate de diméthylaminoéthyle quaternarisé, le chlorure d'ammonium de diallyldiméthyle, ou leurs mélanges. Ces résines cationiques peuvent aussi être à base de polymères naturels hydrosolubles, comme par exemple, les polysaccharides cationiques comme le guar cationique, la cellulose cationique, ou leurs mélanges.
Les compositions cosmétiques faisant l'objet de l'invention peuvent également contenir des dérivés polymériques exerçant une fonction protectrice.
Ces dérivés polymériques peuvent être présents en quantités de l'ordre de 0,01-10%, de préférence environ 0,1-5%, et tout particulièrement de l'ordre de 0,2-3% en poids, dérivés polymériques tels que
. les dérivés cellulosiques tels que les hydroxyéthers de cellulose, la méthylcellulose, l'éthylcellulose, l'hydroxypropyl méthylcellulose, l'hydroxybutyl méthylcellulose
. les polyvinylesters greffés sur des troncs polyalkylenes tels que les polyvinylacétates greffés sur des troncs polyoxyéthylènes (EP-A-219 048)
. les alcools polyvinyliques
. les copolymères polyesters à base de motifs ethylène téréphtalate et/ou propylène téréphtalate et polyoxyéthylène téréphtalate, avec un rapport molaire (nombre de motifs) ethylène téréphtalate et/ou propylène téréphalate / (nombre de motifs) polyoxyéthylène téréphtalate de l'ordre de 1/10 à 10/1, de préférence de l'ordre de 1/1 à 9/1, les polyoxyéthylène téréphtalates présentant des unités polyoxyéthylène ayant un poids moléculaire de l'ordre de 300 à 5000, de préférence de l'ordre de 600 à 5000 (US-A-3 959 230, US-A-3 893 929, US-A-4 116 896, US-A-4 702 857, US-A-4 770 666) ;
. les oligomères polyesters sulfonés obtenus par sulfonation d'un oligomère dérivé de de l'alcool allylique éthoxylé, du diméthyltéréphtalate et du 1,2 propylène diol, présentant de 1 à 4 groupes sulfonés (US-A-4 968 451)
. les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896)
. les polyesters-polyuréthanes obtenus par réaction d'un polyesters de masse moléculaire en nombre de 300-4000 obtenus à partir d'acide adipique et/ou d'acide téréptalique et/ou d'acide sulfoisophtalique et d'un diol de masse inférieure à 300, sur un prépolymère à groupements isocyanates terminaux obtenus à partir d'un polyoxyéthylène glycol de masse moléculaire de 600-4000 et d'un diisocyanate (FR-A-2 334 698)
. les monoamines ou polyamines éthoxylées, les polymères d'amines éthoxylées (US-A-4 597 898, EP-A-11 984)
. les oligomères polyesters sulfonés obtenus par condensation de l'acide isophtalique, du sulfosuccinate de diméthyle et de diéthylène glycol (FR-A-2 236 926)
Les performances des compositions cosmétiques faisant l'objet de l'invention peuvent aussi être améliorées par l'emploi d'agents plastifiants. L'agent plastifiant pourra constituer entre 0.1 à 20% de la formulation de préférence de 1 à 15%. Parmi les agents plastifiants particulièrement utiles, on peut citer les adipates, les phtalates, les isophtalates, les azélates, les stéarates, les copolyols silicones, les glycols, l'huile de ricin, ou leurs mélanges.
Ces compositions cosmétiques peuvent aussi contenir des agents tensioactifs qui servent à disperser, émulsionner, solubiliser, stabiliser divers composés utilisés pour leurs propriétés émollientes ou humectantes. Les agents tensioactifs sont utilisés dans ces compositions à des concentrations variant de 0.05 à 50 % en poids de la préparation. On retrouve ainsi des tensioactifs anioniques, non-ioniques, cationiques, zwitterioniques ou amphotères ou des mélanges de ces tensioactifs.
Parmi ces agents tensio-actifs des
agents tensio-actifs anioniques tels que
. les alkylesters sulfonates de formule R-CH(SO₃M)-COOR', où R représente un radical alkyle en C₈-C₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...). On peut citer tout particulièrement les méthyl ester sulfonates dont les radical R est en C14-C16 ;
. les alkylsulfates de formule ROSO₃M, où R représente un radical alkyle ou hydroxyalkyle en C₁₀-C₂₄, de préférence en C₁₂-C₂₀ et tout particulièrement en C₁₂-C₁₈, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;
. les alkylamides sulfates de formule RCONHR'OSO₃M où R représente un radical alkyle en C₂-C₂₂, de préférence en C₆-C₂₀, R' un radical alkyle en C₂-C₃, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
. les sels d'acides gras saturés ou insaturés en C₈-C₂₄, de préférence en C₁₄-C₂₀, les alkylbenzènesulfonates en C₉-C₂₀, les alkylsulfonates primaires ou secondaires en C₈-C₂₂, les alkylglycérol sulfonates, les acides polycarboxyliques sulfonés décrits dans GB-A-1 082 179, les sulfonates de paraffine, les N-acyl N-alkyltaurates, les alkylphosphates, les alkyliséthionates, les alkylsuccinamates les alkylsulfosuccinates, les monoesters ou diesters de sulfosuccinates, les N-acyl sarcosinates, les sulfates d'alkylglycosides, les polyéthoxycarboxylates
le cation étant un métal alcalin (sodium, potassium, lithium), un reste ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...) ;
agents tensio-actifs non-ioniques tels que
. les alkylphénols polyoxyalkylénés (polyéthoxyéthylénés, polyoxypropylénés, polyoxybutylénés) dont le substituant alkyle est en C₆-C₁₂ et contenant de 5 à 25 motifs oxyalkylènes ; à titre d'exemple, on peut citer les TRITON X-45, X-114, X-100 ou X-102 commercialisés par Rohm & Haas Cy. ;
. les glucosamides, glucamides ;
. les glycérolamides dérivés de N-alkylamines (US-A-5,223,179 et FR-A-1,585,966)
. les alcools aliphatiques en C₈-C₂₂ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy.
. les produits résultant de la condensation de l'oxyde d'éthylène avec un composé hydrophobe résultant de la condensation de l'oxyde de propylène avec le propylène glycol, tels les PLURONIC commercialisés par BASF ;
. les oxydes d'amines tels que les oxydes d'alkyl C₁₀-C₁₈ diméthylamines, les oxydes d'alkoxy C₈-C₂₂ éthyl dihydroxy éthylamines ;
. les alkylpolyglycosides décrits dans US-A-4 565 647 et leurs polyoxyalkylénés;
. les amides d'acides gras en C8-C20
. les acides gras éthoxylés
. les amides, amines, amidoamines éthoxylées
agents tensio-actifs cationiques tels que les halogénures d'alkyldiméthylammonium
agents tensio-actifs amphotères et zwitterioniques tels que
. les alkyl ampho acétates et di-acétates, les alkyl bétaines, les alkyl amidopropyl bétaines, les alkyltriméthylsulfobétaïnes, les produits de condensation d'acides gras et d'hydrolysats de proteines, les alkylampho-propionates ou -dipropionates, les alkylsultaines, les dérivés amphotères des alkylpolyamines comme l'AMPHIONIC XL® commercialisé par RHONE-POULENC, AMPHOLAC 7T/X® et AMPHOLAC 7C/X® commercialisés par BEROL NOBEL sont utilisés pour diminuer l'irritation provoquée par les autres agents tensioactifs, principalement les agents tensioactifs anioniques.
Pour diminuer encore l'irritation ou l'aggression de la peau, on peut aussi rajouter des polymères hydrosolubles ou hydrodispersables comme le collagène ou certains dérivés non allergisants de protéines animales ou végétales (hydrolisats de protéines de blé par exemple), des hydrocolloides naturels (gomme de guar, de caroube, de tara, .....) ou issus de procédés de fermentation comme la gomme xanthane et les dérivés de ces polycarbohydrates comme les celluloses modifiées (par exemple Hydroxyéthylcellulose, carboxyméthylcellulose, celluloses cationiques - les POLYMER JR commercialisés par la société UNION CARBIDE -), les dérivés du guar ou de la caroube comme leurs dérivés cationiques (JAGUAR C13S , JAGUAR C162 commercialisés par RHONE-POULENC) ou des dérivés non-ioniques (par exemple hydroxypropylguar), les dérivés anioniques (carboxyméthylguar) ou les dérivés mixtes non-ioniques/anioniques comme les carboxy-hydroxypropyl-guars ou non-ioniqueslcationiques. On peut aussi ajouter alternativement ou en association des polymères synthétiques comme les polyacrylates ou des polymères cationiques synthétiques, connus sous le nom générique CTFA de "Polyquatemium", par exemple les polymères MIRAPOL A15 ou MIRAPOL 550 de la société RHONE-POULENC.
On peut aussi avantageusement ajouter à ces compositions des agents séquestrants des métaux, plus particulièrement ceux séquestrants du calcium comme les ions citrates ou des agents émollients comme les silicones ou des huiles ou corps gras utilisés à ce propos dans l'industrie cosmétique (huiles minérales, esters d'acides gras, triglycérides, silicones, ....).
A ces ingrédients on rajoute généralement un ou des parfums, des colorants et/ou des agents opacifiants comme des pigments (particules d'oxyde de titane). On peut aussi incorporer dans la composition des agents bactéricides ou fongicides afin d'améliorer la désinfection de la peau comme par exemple le triclosan.
Parmi les agents humectants, on peut citer le glycérol, le sorbitol, l'urée, le collagène, la gélatine, l'aloe vera, l'acide hyaluronique, ... Les émollients sont généralement choisis parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin...) ou les huiles d'origine animale (suif, huiles de poisson,...), des dérivés de ces huiles comme les huiles hydrogénées, les dérivés de la lanoline, les huiles minérales ou les huiles paraffiniques, le perhydrosqualane, le squalène, les diols comme le 1-2-propanediol, le 1-3-butanediol, l'alcool cétylique, l'alcool stéarylique, l'alcool oléique, les polyéthylèneglycols ou polypropyléneglycols, les esters gras comme le palmitate d'isopropyl, le cocoate d'éthyl-2-hexyl, le myristyl myristate, les esters de l'acide lactique, l'acide stéarique, l'acide béhennique, l'acide isostéarique, les huiles silicones regroupant les polydiméthylsiloxanes cycliques, les polydiméthylsiloxanes α-ω hydroxylées, les polydiméthylsiloxanes α-ω triméthylsilyllés, les polyorganosiloxanes comme les polyalkylméthylsiloxanes, les polyinéthylphénylsiloxanes, les polydiphénylsiloxanes, les dérivés aminés des silicones, les cires silicones, les silicones copolyéthers (comme l'huile SILBIONE 70646 commercialisée par la société RHONE-POULENC ou la DC 190 commercialisée par DOW CORNING) ou les dérivés mixtes de silicones incluant différents types de dérivatisation (comme les copolymères mixtes polyalkylméthylsiloxanes-silicones copolyéthers).
A ces composés, on peut ajouter en association des poudres ou des particules minérales comme du carbonate de calcium, des oxydes minéraux sous forme de poudre ou sous forme colloidale (particules de taille inférieure ou de l'ordre de un micromètre, parfois de quelque dizaines de nanomètres) comme du dioxyde de titane, de la silice, des sels d'aluminium utilisés généralement comme antitranspirants, du kaolin, du talc, des argiles et leurs dérivés ...
Des agents conservateurs comme les méthyl, éthyl, propyl et butyl esters de l'acide p-hydroxybenzoïque, le benzoate de sodium, le GERMABEN (nom de marque) ou tout agent chimique évitant la prolifération bactérienne ou des moississures et utilisé traditionnellement des les compositions cosmétiques sont généralement introduits dans ces compositions à hauteur de 0,01 à 3 % en poids. La quantité de ces produits est généralement ajustée pour éviter toute prolifération de bactéries, moississures ou levures dans les compositions cosmétiques.
Alternativement à ces agents chimiques, on peut parfois utiliser des agents modifiants l'activité de l'eau et augmentant fortement la pression osmotique comme les carbohydrates ou des sels.
Pour protéger la peau ou les cheveux des agressions du soleil et des rayons UV, on peut ajouter à ces formulations des filtres solaires qui sont soient des composés chimiques absorbant fortement le rayonnement UV comme les composés autorisés dans la directive Européenne N° 76/768/CEE, ses annexes et les modifications ultérieures de cette directive ou le dioxyde de titane ou les oxydes de cérium sous forme de poudre ou de particules colloïdales. Ces poudres peuvent éventuellement être traitées en surface pour augmenter l'efficacité de leur action anti-UV ou pour faciliter leur incorporation dans les formulations cosmétiques ou pour inhiber la photoréactivité de surface.
Des parfums, des colorants ou des pigments peuvent être ajoutés pour augmenter l'agrément lors de l'utilisation de la composition par le consommateur.
Finalement, la composition peut aussi contenir des polymères viscosants ou gélifiants, comme les polyacrylates réticulés -CARBOPOL commercialisés par GOODRICH-, les dérivés de la cellulose comme l'hydroxypropylcellulose, la carboxyméthylcellulose, les guars et leurs dérivés, la caroube, la gomme de tara ou de cassia, la gomme xanthane, les alginates, les carraghénannes, les dérivés de la chitine comme le chitosan ... utilisés seuls ou en association, ou les mêmes composés, généralement sous la fome de polymères hydrosolubles mdifiés par des groupements hydrophobes liés de manière covalente au squelette polymère comme décrit dans le brevet WO 92/16187 et/ou de l'eau pour amener le total des constituants de la formulation à 100 %.
Les compositions cosmétiques faisant l'objet de l'invention peuvent également contenir des agents dispersants polymériques en quantité de l'ordre de 0,1-7% en poids, pour contrôler la dureté en calcium et magnésium, agents tels que
. les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acryliqiue, acide ou anhydride maleique, acide fumarique, acide itaconique, acide aconitique, acide mesaconique, acide citraconique, acide méthylènemalonique, et tout particulièrement les polyacrylates de masse moléculaire de l'ordre de 2 000 à 10 000 (US-A-3 308 067), les copolymères d'acide arylique et d'anhydride maleique de masse moléculaire de l'ordre de 5 000 à 75 000 (EP-A-66 915)
. les polyéthylèneglycols de masse moléculaire de l'ordre de 1000 à 50 000
Les compositions cosmétiques faisant l'objet de l'invention peuvent aussi contenir tout type de produits visant à modifier de manière temporaire la coloration externe d'une surface corporelle. A titre d'exemples, on peut citer les produits colorants pour le cheveu, les produits de maquillage comme les rouges à lèvres, vernis à ongles, ou produits pour paupières et cils.
Parmi les agents colorants pouvant être utilisés comme constituants des compositions cosmétiques faisant l'objet de l'invention, on peut citer les produits décrits dans l'annexe IV ("list of colouring agents allowed for use in cosmetic products") de la directive européenne n° 76/768/CEE du 27 juillet 1976 dite directive cosmétique.

Les compositions résultant de cette invention peuvent être aussi utilisées dans des formulations de pains de toilettes appelées savonnettes ou savons.
Les compositions classiques de pains de toilette comprennent généralement des sels d'acide gras utilisés en association avec des tensioactifs de l'invention et éventuellement des tensio-actifs autres que les sels d'acides gras ou les acides gras eux-mêmes. Ces compositions peuvent même ne contenir aucun acide gras ou sel d'acide gras et leurs formulations reposent alors sur d'autres tensioactifs comme par exemple les alkyl isethionates de sodium en C₈-C₂₂ ou les alkylsulfates de sodium en C₈-C₂₂.
A ces compositions on peut aussi ajouter différents constituants utiles pour favoriser l'hydratation de la peau comme certains carbohydrates (glycerol, sorbitol par exemple), des polyethylènes glycol ou polypropylène glycol, des dérivés alcoxylés des sucres ou de leurs dérivés (méthyl glucose par exemple), des polymères hydrosolubles ou hydrodispersables comme le collagène ou certains dérivés non allergisants de protéines animales ou végétales (hydrolysats de protéines de blé par exemple), des hydrocolloides naturels (gomme de guar, de caroube, de tara ...) ou issus de procédés de fermentation comme la gomme xanthane et les dérivés de ces polycarbohydrates comme les celluloses modifiées (par exemple Hydroxyéthylcellulose, carboxyméthylcellulose, celluloses cationiques comme les POLYMER JR® commercialisés par la société UNION CARBIDE), les dérivés du guar ou de la caroube comme leurs dérivés cationiques (JAGUAR C13S® , JAGUAR C162® commercialisés par RHONE-POULENC) ou des dérivés non-ioniques (par exemple hydroxypropylguar), les dérivés anioniques (carboxyméthylguar) ou les dérivés mixtes non-ioniques/anioniques comme les carboxy-hydroxypropyl-guars ou non-ioniques/cationiques. On peut aussi ajouter alternativement ou en association des polymères synthétiques comme les polyacrylates ou des polymères cationiques synthétiques, connus sous le nom générique CTFA de "Polyquatemium", par exemple les polymères MIRAPOL A15® ou MIRAPOL 550® de la société RHONE-POULENC. On peut aussi avantageusement ajouter à ces compositions des agents séquestrants des métaux, plus particulièrement ceux séquestrants du calcium comme les ions citrates ou des agents émollients comme les silicones ou des huiles ou corps gras utilisés à ce propos dans l'industrie cosmétique (huiles minérales, esters d'acides gras, triglycérides, silicones, ....).
A ces ingrédients on rajoute généralement un ou des parfums, des colorants et/ou des agents opacifiants comme des pigments (particules d'oxyde de titane). On peut aussi incorporer dans la composition des agents bactéricides ou fongicides afin d'améliorer la désinfection de la peau.
Dans un pain de toilette dont la formulation est constituée principalement de savons d'acides gras monocarboxyliques (sels de sodium, potassium, mono-, di- ou tri-éthanolammonium) ; les teneurs en savons d'acides gras sont généralement de plus de 25ù en poids de la formulation, généralement de 30 à 95% en poids.
Dans un pain de toilette dont la formulation repose sur d'autres constituants principaux que les savons d'acides gras, on trouve dans la formulation de 0 à 50 % en poids, préférentiellement de 1 à 40 % en poids de ces savons d'acides gras.
Ces compositions de pains de toilettes peuvent aussi contenir de 0 à 95%, de préférence de 0 à 60% d'agents tensioactifs autres que des savons, notamment les C₈-C₂₂ alkyl ou alkényl iséthionates,de même que les compositions résultant de cette invention, les alkylampho -propionates ou -dipropionates.
De 1 à 15% d'acides gras libres en C₈-C₂₂ peuvent aussi être introduits dans les compositions de savons en tant qu'agents surgraissants ou pour modifier l'aspect et le caractère crémeux de la mousse lors du lavage.
On peut aussi retrouver dans ces compositions des cires comme des cires paraffiniques, des cires naturelles comme la cire d'abeille ou l'ozokérite ou des cires silicones. Ces cires sont avantageusement utilisées pour améliorer l'aspect, la tenue, la processabilité et la conservation au stockage des pains de toilettes.
Les exemples suivants sont donnés à titre illustratif.

### Exemple 1 :

Dans un réacteur en acier inoxydable de 7,5 litres, muni d'un agitateur à ancre, d'une double enveloppe pour la circulation d'un liquide caloporteur et d'une colonne de distillation régulée par une électrovanne, on introduit à froid :
- 2363 g (12,17 moles) de téréphtalate de diméthyle (DMT)
- 590 g (1,99 mole) de sulfo-oxysulfonyl-5 isophtalate de diméthyle (SIPD)
- 2464 g (39,7 moles) d'éthylène glycol (EG)
- 1,34 g d'orthotitanate de butyle (TBOT) comme catalyseur d'interéchange et de polycondensation.
Le rapport molaire diols/diesters RM1 est de 2,80.
L'agitation est mise en marche, puis le contenu du réacteur est porté rapidement à 182°C, température à laquelle le méthanol commence à distiller. La température du mélange réactionnel est alors portée à 220°C/230°C en environ 120 minutes pour distiller l'ensemble du méthanol et une partie de l'éthylène glycol en excès. Lorsque la masse réactionnelle atteint 230°C, on introduit, sur une période de 1 heure la suspension acide isophtalique/éthylène glycol suivante :
- 497 g (2,99 moles) d'acide isophtalique (AI)
- 497 g (8,00 moles) d'éthylène glycol (EG).
Le rapport molaire diols/diacides RM2 est de 2,67.
Pendant l'introduction, la température de la masse réactionnelle ne chute pas au-dessous de 227°C et ne dépasse pas 233°C.
Dès que l'introduction de la suspension précédente est terminée, sont également introduits dans le réacteur 70 g (soit 2 % en poids par rapport à la quantité théorique de polyester non siliconé formé par mise en oeuvre des diesters, diacides et diols ci-dessus) de l'oligomère (S1) constitué de polydiméthylsiloxane α-ωbispropyloxybenzoate de méthyle de poids moléculaire de l'ordre de 6 400, les motifs propyloxybenzoate de méthyle liés aux atomes de silicium en *α* et ω ayant pour formule 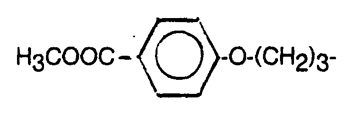 La masse réactionnelle est alors portée de 230°C à 250°C en 1 heure. Durant cette période, on distille un mélange eau / éthylène glycol / méthanol. A 250°C, la masse réactionnelle est transférée dans un autoclave préchauffé à 250°C. La pression est alors réduite de 1 013 millibars à 1 millibar en 60 minutes et la polycondensation est poursuivie pendant environ 40 à 45 minutes, toujours à 250°C et avec un vide poussé qui oscille entre 0,5 et 1 millibar.
Le polymère est ensuite coulé et refroidi.
On réalise les analyses suivantes :
1. Evaluation de la masse molaire par chromatographie par perméation de gel (GPC) dans le diméthyle acétamide /LiCl à 20°C. Les valeurs sont données en équivalents polystyrène.
2. Evaluation de la teneur en silicium dans le polymère par minéralisation par voie sèche en présence de carbonates alcalins et analyse par spectrométrie d'émission atomique générée par plasma à couplage inductif de la solution du silicate alcalin obtenu précédemment.
3. Turbidité en dispersion aqueuse à 25% en poids d'extrait sec, mesurée à l'aide d'un turbidimètre RATIO/XR (modèle 43900 "HACH").
4. Mesure du taux de soufre
Les caractéristiques du copolyester sulfoné à motifs polyorganosiloxanes préparé sont données au tableau 1.

### Exemple 2

On reproduit l'exemple 1 en suivant le même mode opératoire, mais en remplaçant l'oligomère (S1) par la même quantité d'oligomère (S2) constitué d'α-ω bis(hydroxy)polydiméthylsiloxane de poids moléculaire de l'ordre de 11 000.
Les caractéristiques du copolyester sulfoné à motifs polyorganosiloxanes préparé sont données au tableau 1.

### Exemple 3

On reproduit l'exemple 2 en introduisant 140 g de l'oligomère S2 (4%) au lieu de 70g.
Les caractéristiques du copolyester sulfoné à motifs polyorganosiloxanes préparé sont données au tableau 1.

### Exemple 4

On reproduit l'exemple 2 en introduisant 280 g de l'oligomère S2 (8 %) au lieu de 70g.
Les caractéristiques du copolyester sulfoné à motifs polyorganosiloxanes préparé sont données au tableau 1.

### Exemple 5

On reproduit l'exemple 1 en suivant le même mode opératoire, mais en remplaçant les 2% d'oligomère (S1) par 4% (140g) d'oligomère (S3) constitué d'α-ω bis(hydroxypropyl)polydiméthylsiloxane de poids moléculaire voisin de 6 400, les motifs hydroxypropyl liés aux atomes de silicium en α et ω ayant pour formule -(CH₂)₃-OH.
Les caractéristiques du copolyester sulfoné à motifs polyorganosiloxanes préparé sont données au tableau 1.

**Tableau 1**

| Exemple | Oligomère | % Oligomère introduit | % Si dosé | % S | Mn | Mw | Turbidité (NTU) |
|---|---|---|---|---|---|---|---|
| 1 | S1 | 2 | 0.47 | 1,63 | | | 172 |
| 2 | S2 | 2 | 0.49 | 1,63 | | | 450 |
| 3 | S2 | 4 | 1.00 | 1,66 | 29600 | 55800 | 810 |
| 4 | S2 | 8 | 2.32 | 1,7 | 26000 | 53600 | >2000 |
| 5 | S3 | 4 | 1.15 | 1,66 | 28800 | 53330 | 760 |

### Exemple 6 : composition d'un spray fixant pour cheveux

| **composant** | **% poids** |
|---|---|
| produit de l'un des exemples 1 -5 | 3 |
| éthanol | 75 |
| parfum | 0,1 |
| gaz propulseur | qsp 100 |

### Exemple 7 : shampoing structurant

| **composant** | % **poids** |
|---|---|
| agent structurant | |
| produit de l'un des exemples 1-5 | 2 |
| octaméthyl cyclotétra siloxane | 2 |

| premix | |
|---|---|
| gomme silicone | 0,5 |
| dimethicone | 0,5 |

| mix principal | |
|---|---|
| lauryl sulfate d'ammonium | 11 |
| coco ampho diacétate | 2 |
| éthylène glycol distéarate | 1 |
| gomme xanthane | 1 |
| conservateur | 1,2 |
| acide citrique qsp pH 6.5 | qsp pH 6,5 |
| eau distillée | qsp 100 |

L'agent structurant et le prémix sont préparés séparemment de manière conventionnelle. Le Mix principal est préparé en dissolvant d'abord la gomme xanthane dans l'eau. Puis les autres composés sont ajoutés et le mix principal chauffé sous agitation à 80°C pendant 30 minutes. L'agent structurant et le prémix sont alors ajoutés de manière séquentielle, puis on laisse le mélange résiduel revenir à température ambiante sous agitation.

### Exemple 8 : shampoing

| **composant** | **% poids** |
|---|---|
| lauryl sulfate d'ammonium | 7 |
| lauryl ether sulfate d'ammonium | 5 |
| coco ampho diacétate | 4 |
| produit de l'un des exemples 1-5 | 1 |
| hydroxypropyl guar | 1 |
| conservateur | 0,4 |
| eau distillée | qsp 100 |

### Exemple 9 : shampoing conditionneur

| **composant** | **% poids** |
|---|---|
| agent structurant | |
| produit de l'un des exemples 1-5 | 1 |
| phenyl pentaméthyl disiloxane | 4 |

| prémix silicone | |
|---|---|
| gomme silicone | 0,3 |
| octa méthyl tétrasiloxane | 1,7 |

| mix principal | |
|---|---|
| alcools cétylique/stéarique | 1,7 |
| guar hydroxypropyl trimonium chlorure | 0,85 |
| hydroxypropyl guar | 0,5 |
| ceteareth-20 | 0,35 |
| parfum | 0,2 |
| diméthicone copolyol | 0,2 |
| conservateur | 0,04 |
| eau distillée | qsp 100 |

### Exemple 10: gel coiffant

| **composant** | **% poids** |
|---|---|
| produit de l'un des exemples 1-5 | 2 |
| hydroxypropyl guar | 0,75 |
| triéthanolamine | 1 |
| colorant | 0,05 |
| parfum | 0,1 |
| laureth-23 | 0,1 |
| eau distillée | qsp 100 |

### Exemple 11 : gel douche conditionneur

| **Composant** | **CTFA** | % p/p |
|---|---|---|
| eau distillée | eau | qsp 100 |
| EMPICOL ESB | Sodium Laureth-2 Sulfate | 25,0 |
| MIRANOL ULTRA C32 | Sodium Cocoamphoacetate | 10,0 |
| GEROPON TC42 | Sodium Methyl Cocoyl Taurate | 12,0 |
| produit de l'un des exemples 1-5 | | 0,3 |
| Glycerol | Glycerol | 1,0 |
| SILBIONE 71834 | Diphenyldimethicone emulsion | **2,00** |
| MIRASHEEN 202 | | 8,0 |
| Germaben II | | 0,20 |
| Fragance (PARFEX 49902) | | 0,20 |
| Citric Acid | Citric Acid | qsp pH 6,5 |
| NaCI | sodium chloride | qsp* |

| | | |
|---|---|---|
| qsp * : qsp pour obtenir une viscosité de 4000 mPa.s à 25°C | | |

### Exemple 12 : mousse coiffante

| **composant** | **% p/p** |
|---|---|
| eau | qsp 100 |
| produit de l'un des exemples 1-5 | 3 |
| coco ampho diacétate | 0,5 |
| sodium méthyl oleyl taurate | 2 |
| DMDM hydantoine | 0,8 |
| alcool isostéarique éthoxylé | 0,1 |
| parfum | 0,1 |
| gaz propulseur | 7 |
| EDTA disodique | 0,2 |

### Exemple 13 : composition anti-acnée

| **composant** | **% p/p** |
|---|---|
| eau | qsp 100 |
| acide salicylique | 2 |
| produit de l'un des exemples 1-5 | 2 |
| éthanol | 40 |

## Revendications

1. Composition cosmétique pour le cheveu et/ou la peau, **caractérisée en ce qu**'elle comprend au moins un copolyester sulfoné hydrodispersable à motifs polyorganosiloxanes comportant une pluralité de motifs récurrents polyesters sulfonés et de motifs polyorganosiloxanes, ledit copolyester sulfoné à motifs polyorganosiloxanes présentant
. une masse moléculaire en nombre de l'ordre de 5 000 à 45 000, de préférence de l'ordre de 8 000 à 35 000,
. une teneur pondérale en soufre de l'ordre de 0,5 à 10 %, de préférence de l'ordre de 1 à 8 %, par rapport auxdits copolyesters sulfonés à motifs polyorganosiloxanes
. et une teneur en silicium de l'ordre de 0,05 à 20 %, de préférence de l'ordre de 0,1 à 10 %, tout particulièrement de l'ordre de 0,1 à 5 % en poids par rapport auxdits copolyesters sulfonés à motifs polyorganosiloxanes.

2. Composition cosmétique selon la revendication 1), **caractérisée en ce que** ledit copolyester sulfoné hydrodispersable à motifs polyorganosiloxanes est choisi parmi les copolyesters sulfonés hydrodispersables à motifs polyorganosiloxanes susceptibles d'être obtenus par polymérisation (estérification et/ou transestérification et polycondensation) d'une composition monomère (M) à base :
- d'au moins un monomère acide dicarboxylique aromatique non sulfoné (A), son anhydride ou un de ses diesters,
- d'au moins un monomère diol aliphatique ou cycloaliphatique (D),
- d'au moins un monomère sulfoné (MS) choisi parmi les acides dicarboxyliques aromatiques sulfonés ou aliphatiques sulfonés (MAS), leurs anhydrides ou leurs diesters et les diols aliphatiques sulfonés ou aromatiques sulfonés (MDS),
les quantités relatives des monomères, sulfonés ou non, à fonctions diacides, diesters ou anhydride, et des monomères, sulfonés ou non, à fonctions diols, correspondant à un rapport nombre de fonctions OH de la composition monomère / nombre de fonctions ou d'équivalents fonctions COOH de la composition monomère de l'ordre de 1,05 à 4, de préférence de l'ordre de 1,1 à 3,5, tout particulièrement de l'ordre de 1,8 à 3 ;
ladite opération de polymérisation étant réalisée en présence d'au moins un polydiorganosiloxane réactif de formule (I) 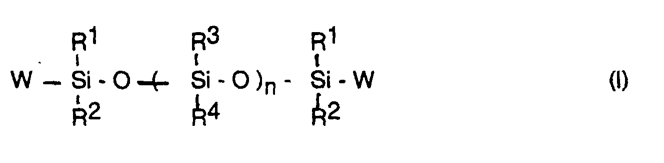 formule dans laquelle
- les symboles R¹, R², R³ et R⁴, identiques ou différents, représentent des radicaux alkyles linéaires ou ramifiés en C₁-C₁₆, de préférence en C₁-C₄, cycloalkyles en C₅-C₁₅, de préférence en C₆-C₈, phényle, alkylphényle avec une partie alkyle en C₁-C₄,
- W représente un groupement susceptible de réagir avec au moins un des monomères de la composition monomère (M) dans les conditions de l'opération de polymérisation,
- n est un nombre entier ou décimal de l'ordre de 5 à 15 000, de préference de l'ordre de 30 à 300, et tout particulièrement de l'ordre de 50 à 200 ;
les quantités de monomère sulfoné (MS) et de polyorganosiloxane, ainsi que les conditions de polymérisation étant telles que lesdits copolyesters sulfonés à motifs polyorganosiloxanes obtenus présentent
. une masse moléculaire en nombre de l'ordre de 5 000 à 45 000, de préférence de l'ordre de 8 000 à 35 000,
. une teneur pondérale en soufre de l'ordre de 0,5 à 10 %, de préférence de l'ordre de 1 à 8 %, par rapport auxdits copolyesters sulfonés à motifs polyorganosiloxanes,
. et une teneur en silicium de l'ordre de 0,05 à 20 %, de préférence de l'ordre de 0,1 à 10 %, tout particulièrement de l'ordre de 0,1 à 5 % en poids par rapport auxdits copolyesters sulfonés à motifs polyorganosiloxanes.

3. Composition cosmétique selon la revendication 2), **caractérisée en ce que** le monomère diacide non sulfoné (A) est constitué par de l'ordre de 0 à 100% molaire, de préférence de l'ordre de 50 à 100% molaire, tout particulièrement de l'ordre de 70 à 90% molaire, d'acide téréphtalique et/ou isophtalique et/ou 2,6-naphtalène dicarboxylique sous forme d'un de ses (leurs) diesters inférieurs de méthyle, éthyle, propyle, isopropyle ou butyle et de l'ordre de 100 à 0% molaire, de préférence de l'ordre de 50 à 0% molaire, tout particulièrement de l'ordre de 30 à 10% molaire, d'acide ou anhydride isophtalique et/ou 2,6-naphtalène dicarboxylique et/ou terephtalique.

4. Composition cosmétique selon la revendication 3), **caractérisée en ce que** ledit monomère non sulfoné (A) est constitué par 50 à 90% molaire, de préférence de l'ordre de 70 à 90% molaire, d'acide téréphtalique sous forme d'un de ses diesters inférieurs de méthyle, éthyle, propyle, isopropyle, ou butyle et de l'ordre de 50 à 10% molaire, de préférence de l'ordre de 30 à 10% molaire, d'acide ou anhydride isophtalique.

5. Composition cosmétique selon l'une quelconque des revendications 2) à 4), **caractérisée en ce que** le monomère diol (D) est le monoéthylène glycol et/ou le diéthylène glycol.

6. Composition cosmétique selon l'une quelconque des revendications 2) à 5), **caractérisée en ce que** le monomère diacide sulfoné (MAS) est l'acide ou l'anhydrides sulfo-isophtalique ou sulfo-succinique ou leur diester de méthyle, et de préférence le sodio-oxysulfonyl-5 isophtalate de diméthyle.

7. Composition cosmétique selon l'une quelconque des revendications 2) à 6), **caractérisée en ce que** ledit monomère diacide sulfoné (MAS) est mis en oeuvre en quantité correspondant à un rapport molaire (MAS) / (A)+(MAS) de l'ordre de 5/100 à 40/100, de préférence de l'ordre de 7/100 à 35/100, tout particulièrement de l'ordre de 10/100 à 15/100.

8. Composition cosmétique selon l'une quelconque des revendications 2) à 7), **caractérisée en ce que** les polydiorganosiloxanes réactifs mis en oeuvre, sont ceux de formule (I), dans laquelle W représente une fonction OH ou un groupement -A-Z, où :
. A représente un radical hydrocarboné divalent lié à l'atome de silicium vicinal par une liaison Si-C ou Si-O-C, pouvant éventuellement comporter un hétéroatome pris dans le groupe formé par O, Si, N et S
. Z représente un groupement susceptible de réagir avec au moins un des monomères ci-dessus dans les conditions de polymérisation.

9. Composition cosmétique selon l'une quelconque des revendications 2) à 8), **caractérisée en ce que** les motifs réactifs W sont
| | |
|---|---|
| -OH | |
| -(CH₂)₂-OH | -(CH₂)₃-OH |
| -(CH₂)₂-O-(CH₂)₂-OH | -(CH₂)₃-O-Φ-OH |
| -(CH₂)₂-COOCH₃ | -Φ -COOCH₃ |
| -(CH₂)₃-Φ-COOCH₃ | -(CH₂)₃-O-Φ-COOCH₃ |
| -(CH₂)₃-O-(CH₂)₂-OH | -(CH₂)₃-O-CH₂-CH(CH₃)OH |

10. Composition cosmétique selon l'une quelconque des revendications 1) à 9), **caractérisée en ce qu**'elle comprend
- de l'ordre de 0,1 à 50%, de préférence de l'ordre de 0,1 à 5% de son poids dudit copolyester sulfoné à motifs polyorganosiloxanes
- et de l'ordre de 0,5 à 99,5%, de préférence de l'ordre de 5 à 99,5% de son poids d'un véhicule compatible avec le cheveu et/ou la peau.

11. Composition cosmétique selon l'une quelconque des revendications 1) à 10), sous forme de formule de rinçage, lotion, shampoing, conditionneur pour cheveux, mousse ou gel pour le coiffage ou le peignage des cheveux, lotion pour les mains ou le corps, lait de toilette, composition démaquillante, produit régulant l'hydratation de la peau, crème de soin, crème ou lait de protection contre le soleil et le rayonnement ultraviolet, préparation anti-acnée, analgésique local, mascara, pain de toilette, savonnette.

## Patentansprüche

1. Kosmetische Zusammensetzung für das Haar und/oder die Haut, **dadurch gekennzeichnet**, dass diese mindestens einen in Wasser dispergierbaren sulfonierten Copolyester mit Polyorganosiloxanmotiven, der eine Mehrzahl von sulfonierten Polyester-Struktureinheiten und von Polyorganosiloxanmotiven umfasst, enthält, wobei der sulfonierte Copolyester mit Polyorganosiloxanmotiven:
. eine zahlenmäßige Molekülmasse in der Größenordnung von 5000 bis 45000, vorzugsweise in der Größenordnung von 8000 bis 35000,
. einen auf das Gewicht bezogenen Schwefelgehalt in der Größenordnung von 0,5 bis 10%, vorzugsweise in der Größenordnung von 1 bis 8% bezogen auf die sulfonierten Copolyester mit Polyorganosiloxanmotiven
. und einen Siliciumgehalt in der Größenordnung von 0,05 bis 20 Gew.-%, vorzugsweise in der Größenordnung von 0,1 bis 10 Gew.-%, insbesondere in der Größenordnung von 0,1 bis 5 Gew.-% bezogen auf die sulfonierten Copolyester mit Polyorganosiloxanmotiven
aufweist.

2. Kosmetische Zusammensetzung nach Anspruch 1, **dadurch gekennzeichnet**, dass der in Wasser dispergierbare sulfonierte Copolyester mit Polyorganosiloxanmotiven ausgewählt wird aus den in Wasser dispergierbaren sulfonierten Copolyestern mit Polyorganosiloxanmotiven, die erhalten werden können durch Polymerisation (Veresterung und/oder Umesterung und Polykondensation) einer Monomerzusammensetzung (M) auf Basis von:
- mindestens einem nicht sulfonierten aromatischen Dicarbonsäuremonomer (A), dessen Anhydrid oder einem seiner Diester,
- mindestens einem aliphatischen oder cycloaliphatischen Diolmonomer (D),
- mindestens einem sulfonierten Monomer (MS), ausgewählt aus den sulfonierten aromatischen oder sulfonierten aliphatischen Dicarbonsäuren (MAS), deren Anhydriden oder deren Diestern und den sulfonierten aliphatischen oder sulfonierten aromatischen Diolen (MDS),
wobei die relativen Mengen der gegebenenfalls sulfonierten Monomere mit Disäure-, Diester- oder Anhydridfunktionen und der gegebenenfalls sulfonierten Monomere mit Diolfunktionen einem Verhältnis Anzahl von OH-Funktionen der Monomerzusammensetzung / Anzahl von COOH-Funktionen oder von zu COOH äquivalenten Funktionen der Monomerzusammensetzung in der Größenordnung von 1,05 bis 4, vorzugsweise in der Größenordnung von 1,1 bis 3,5, insbesondere in der Größenordnung von 1,8 bis 3 entsprechen, wobei der Polymerisationsschritt ausgeführt wird in Gegenwart von mindestens einem Polydiorganosiloxanreagens der Formel (I) 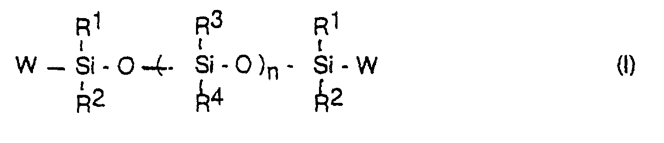 in welcher Formel
- die Symbole R¹, R², R³ und R⁴, die gleich oder verschieden sind, für lineare oder verzweigte C₁-C₁₆-Alkylreste, vorzugsweise C₁-C₄-Alkylreste, C₅-C₁₅-, vorzugsweise C₆-C₈-Cycloalkylreste, Phenyl, Alkylphenyl mit einem C₁-C₄-Alkylteil stehen,
- W für eine Gruppe steht, die mit mindestens einem der Monomere der Monomerzusammensetzung (M) unter den Bedingungen des Polymerisationsschritts reagieren kann,
- n eine ganze Zahl oder Dezimalzahl in der Größenordnung von 5 bis 15000, vorzugsweise in der Größenordnung von 30 bis 300 und insbesondere in der Größenordnung von 50 bis 200 ist, wobei die Mengen an sulfoniertem Monomer (MS) und an Polyorganosiloxan wie auch die Polymerisationsbedingungen so sind, dass die erhaltenen sulfonierten Copolyester mit Polyorganosiloxanmotiven
. eine zahlenmäßige Molekülmasse in der Größenordnung von 5000 bis 45000, vorzugsweise in der Größenordnung von 8000 bis 35000,
. einen auf das Gewicht bezogenen Schwefelgehalt in der Größenordnung von 0,5 bis 10%, vorzugsweise in der Größenordnung von 1 bis 8% bezogen auf die sulfonierten Copolyester mit Polyorganosiloxanmotiven
. und einen Siliciumgehalt in der Größenordnung von 0,05 bis 20 Gew.-%, vorzugsweise in der Größenordnung von 0,1 bis 10 Gew.-%, insbesondere in der Größenordnung von 0,1 bis 5 Gew.-% bezogen auf die sulfonierten Copolyester mit Polyorganosiloxanmotiven
aufweisen.

3. Kosmetische Zusammensetzung nach Anspruch 2, **dadurch gekennzeichnet**, dass das nicht-sulfonierte Disäuremonomer (A) in der Größenordnung von 0 bis 100 mol-%, vorzugsweise in der Größenordnung von 50 bis 100 mol-%, insbesondere in der Größenordnung von 70 bis 90 mol-% aus Terephthalsäure und/oder Isophthalsäure und/oder 2,6-Naphthalindicarbonsäure in Form von einem ihrer niederen Methyl-, Ethyl-, Propyl-, Isopropyl- oder Butyldiester und in der Größenordnung von 100 bis 0 mol-%, vorzugsweise in der Größenordnung von 50 bis 0 mol-%, insbesondere in der Größenordnung von 30 bis 10 mol-% aus Isophthalsäure und/oder 2,6-Naphthalindicarbonsäure und/oder Terephthalsäure oder deren Anhydrid(en) gebildet wird.

4. Kosmetische Zusammensetzung nach Anspruch 3, **dadurch gekennzeichnet**, dass das nicht-sulfonierte Monomer (A) aus 50 bis 90 mol-%, vorzugsweise in der Größenordnung von 70 bis 90 mol-%, Terephthalsäure in Form von einem ihrer niederen Methyl-, Ethyl-, Propyl-, Isopropyl - oder Butyldiester und in der Größenordnung von 50 bis 10 mol-%, vorzugsweise in der Größenordnung von 30 bis 10 mol-% aus Isophthalsäure oder Isophthalsäureanhydrid gebildet wird.

5. Kosmetische Zusammensetzung nach einem der Ansprüche 2 bis 4, **dadurch gekennzeichnet**, dass das Diolmonomer (D) Monoethylenglykol und/oder Diethylenglykol ist.

6. Kosmetische Zusammensetzung nach einem der Ansprüche 2 bis 5, **dadurch gekennzeichnet**, dass das sulfonierte Disäuremonomer (MAS) Sulfoisophthalsäure oder Sulfosuccinsäure oder deren Anhydride oder deren Methyldiester und vorzugsweise Natriumoxysulfonyl-5-isophthalsäuredimethylester ist.

7. Kosmetische Zusammensetzung nach einem der Ansprüche 2 bis 6, **dadurch gekennzeichnet**, dass das sulfonierte Disäuremonomer (MAS) in einer Menge entsprechend einem Molverhältnis (MAS) / (A) + (MAS) in der Größenordnung von 5/100 bis 40/100, bevorzugt in der Größenordnung von 7/100 bis 35/100, insbesondere in der Größenordnung von 10/100 bis 15/100 eingesetzt wird.

8. Kosmetische Zusammensetzung nach einem der Ansprüche 2 bis 7, **dadurch gekennzeichnet**, dass die eingesetzten Polydiorganosiloxanreagenzien jene der Formel (I) sind, in der W für eine OH-Funktion oder eine Gruppe -A-Z steht, worin:
. A für einen an das vicinale Siliciumatom durch eine Si-C- oder Si-O-C-Bindung gebundenen zweiwertigen kohlenwasserstoffhaltigen Rest, der gegebenenfalls ein aus der Gruppe, gebildet aus O, Si, N und S, ausgewähltes Heteroatom umfassen kann, steht,
. Z für eine Gruppe steht, die mit mindestens einem der obigen Monomere unter den Polymerisationsbedingungen reagieren kann.

9. Kosmetische Zusammensetzung nach einem der Ansprüche 2 bis 8, **dadurch gekennzeichnet**, dass die reaktiven Motive W
| | |
|---|---|
| -OH | |
| -(CH₂)₂-OH | -(CH₂)₃-OH |
| -(CH₂)₂-O-(CH₂)₂-OH | -(CH₂)₃-O-Φ-OH |
| -(CH₂)₂-COOCH₃ | -Φ -COOCH₃ |
| -(CH₂)₃-Φ-COOCH₃ | -(CH₂)₃-O-Φ-COOCH₃ |
| -(CH₂)₃-O-(CH₂)₂-OH | -(CH₂)₃-O-CH₂-CH(CH₃)OH |
sind.

10. Kosmetische Zusammensetzung nach einem der Ansprüche 1 bis 9, **dadurch gekennzeichnet**, dass sie umfasst:
- in der Größenordnung von 0,1 bis 50%, vorzugsweise in der Größenordnung von 0,1 bis 5% ihres Gewichtes den sulfonierten Copolyester mit Polyorganosiloxanmotiven
- und in der Größenordnung von 0,5 bis 99,5%, vorzugsweise in der Größenordnung von 5 bis 99,5% ihres Gewichtes einen mit den Haaren und/oder der Haut verträglichen Träger.

11. Kosmetische Zusammensetzung nach einem der Ansprüche 1 bis 10 in Form einer Spülungsformulierung, einer Lotion, eines Shampoos, einer Konditioner-Formulierung für die Haare, eines Schaums oder Gels für das Frisieren oder Kämmen der Haare, einer Lotion für die Hände oder den Körper, einer Waschmilch, einer Abschminkzusammensetzung, eines Produkts, das die Hydratation der Haut reguliert, einer Pflegecreme, einer Creme oder Milch für den Schutz gegen die Sonne und die ultraviolette Strahlung, einer Anti-Akne-Zubereitung, eines lokal wirkenden Analgetikums, einer Maskara, eines Waschstücks, einer Toilettenseife.

## Claims

1. Cosmetic hair and/or skin composition, **characterized in that** it comprises at least one water-dispersible sulphonated copolyester containing polyorganosiloxane units containing a plurality of repeating sulphonated polyester units and polyorganosiloxane units, the said sulphonated copolyester containing polyorganosiloxane units having
· a number-average molecular mass from about 5000 to 45,000, preferably from about 8000 to 35,000,
· a weight content of sulfur from about 0.5 to 10%, preferably from about 1 to 8%, relative to the said sulphonated copolyesters containing polyorganosiloxane units, and
· a silicon content from about 0.05 to 20%, preferably from about 0.1 to 10%, most particularly from about 0.1 to 5%, by weight relative to the said sulphonated copolyesters containing polyorganosiloxane units.

2. Cosmetic composition according to Claim 1, **characterized in that** the said water-dispersible sulphonated copolyester containing polyorganosiloxane units is chosen from water-dispersible sulphonated copolyesters containing polyorganosiloxane units which can be obtained by polymerization (esterification and/or transesterification and polycondensation) of a monomer composition (M) based on :
- at least one non-sulphonated aromatic dicarboxylic acid monomer (A), its anhydride or one of its diesters,
- at least one aliphatic or cycloaliphatic diol monomer (D),
- at least one sulphonated monomer (SM) chosen from sulphonated aromatic or sulphonated aliphatic dicarboxylic acids (SAM), their anhydrides or their diesters and sulphonated aliphatic or sulphonated aromatic diols (SDM),
the relative amounts of the sulphonated or non-sulphonated monomers containing diacid, diester or anhydride functions, and of the sulphonated or non-sulphonated monomers containing diol functions corresponding to a ratio: number of OH functions in the monomer composition/number of COOH functions or equivalent functions in the monomer composition, from about 1.05 to 4, preferably from about 1.1 to 3.5, most particularly from about 1.8 to 3;
the said polymerization operation being carried out in the presence of at least one polydiorganosiloxane reagent of formula (I) 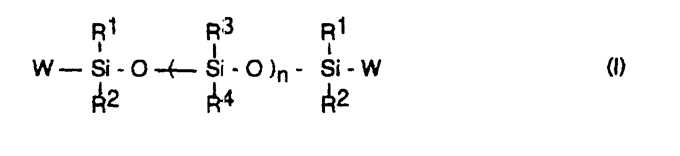 in which formula
- the symbols R¹, R², R³ and R⁴, which may be identical or different, represent linear or branched C₁-C₁₆, preferably C₁-C₄, alkyl radicals, C₅-C₁₅, preferably C₆-C₈, cycloalkyl radicals, phenyl radicals or alkylphenyl radicals with a C₁-C₄ alkyl part,
- W represents a group which can react with at least one of the monomers of the monomer composition (M) under the conditions of the polymerization operation,
- n is an integer or decimal number from about 5 to 15,000, preferably from about 30 to 300 and most particularly from about 50 to 200;
the amounts of sulphonated monomer (SM) and of polyorganosiloxane, as well as the polymerization conditions, being such that the said sulphonated copolyesters containing polyorganosiloxane units which are obtained have
· a number-average molecular mass from about 5000 to 45,000, preferably from about 8000 to 35,000,
· a weight content of sulfur from about 0.5 to 10%, preferably from about 1 to 8%, relative to the said sulphonated copolyesters containing polyorganosiloxane units, and
· a silicon content from about 0.05 to 20%, preferably from about 0.1 to 10%, most particularly from about 0.1 to 5%, by weight relative to the said sulphonated copolyesters containing polyorganosiloxane units.

3. Cosmetic composition according to Claim 2, **characterized in that** the non-sulphonated diacid monomer (A) consists of about 0 to 100 mol%, preferably from about 50 to 100 mol%, most particularly from about 70 to 90 mol%, of terephthalic acid and/or isophthalic acid and/or 2,6-naphthalenedicarboxylic acid in the form of one of its (their) lower diesters of methyl, ethyl, propyl, isopropyl or butyl and from about 100 to 0 mol%, preferably from about 50 to 0 mol%, most particularly from about 30 to 10 mol%, of isophthalic and/or 2,6-naphthalenedicarboxylic and/or terephthalic acid or anhydride.

4. Cosmetic composition according to Claim 3, **characterized in that** the said non-sulphonated monomer (A) consists of 50 to 90 mol%, preferably from about 70 to 90 mol%, of terephthalic acid in the form of one of its lower diesters of methyl, ethyl, propyl, isopropyl or butyl and from about 50 to 10 mol%, preferably from about 30 to 10 mol%, of isophthalic acid or anhydride.

5. Cosmetic composition according to any one of Claims 2 to 4, **characterized in that** the diol monomer (D) is monoethylene glycol and/or diethylene glycol.

6. Cosmetic composition according to any one of Claims 2 to 5, **characterized in that** the sulphonated diacid monomer (SAM) is sulfoisophthalic or sulfosuccinic acid or anhydride or their methyl diester, and preferably dimethyl sodio-5-oxysulfonylisophthalate.

7. Cosmetic composition according to any one of Claims 2 to 6, **characterized in that** the said sulphonated diacid monomer (SAM) is used in an amount corresponding to an (SAM)/(A)+(SAM) molar ratio from about 5/100 to 40/100, preferably from about 7/100 to 35/100, most particularly from about 10/100 to 15/100.

8. Cosmetic composition according to any one of Claims 2 to 7, **characterized in that** the reactive polydiorganosiloxanes used are those of formula (I), **characterized in that** W represents an OH function or a group -A-Z, where:
· A represents a divalent hydrocarbon-based radical attached to the vicinal silicon atom via an Si-C or Si-O-C bond which can optionally contain a hetero atom taken from the group formed by O, Si, N and S,
· Z represents a group which can react with at least one of the above monomers under the polymerization conditions.

9. Cosmetic composition according to any one of Claims 2 to 8, **characterized in that** the reactive units W are
| | |
|---|---|
| -OH | |
| -(CH₂)₂-OH | -(CH₂)₃-OH |
| -(CH₂)₂-O-(CH₂)₂-OH | -(CH₂)₃-O-Φ-OH |
| -(CH₂)₂-COOCH₃ | -Φ -COOCH₃ |
| -(CH₂)₃-Φ-COOCH₃ | -(CH₂)₃-O-Φ-COOCH₃ |
| -(CH₂)₃-O-(CH₂)₂-OH | -(CH₂)₃-O-CH₂-CH(CH₃)OH |

10. Cosmetic composition according to any one of Claims 1 to 9, **characterized in that** it comprises
- from about 0.1 to 50%, preferably from about 0.1 to 5%, of its weight of the said sulphonated copolyester containing polyorganosiloxane units, and
- from about 0.5 to 99.5%, preferably from about 5 to 99.5%, of its weight of a vehicle which is compatible with the hair and/or the skin.

11. Cosmetic composition according to any one of Claims 1 to 10, in the form of a rinsing formula, a lotion, a shampoo or a conditioner for the hair, a mousse or gel for styling or combing the hair, a hand or body lotion, a cleansing milk, a make-up-removing composition, a product for regulating skin moisturization, a care cream, a cream or milk for protecting against sunlight and ultraviolet radiation, an anti-acne preparation, a local analgesic, a mascara, a toiletry bar or a soap.
